# EUROPÄISCHE PATENTSCHRIFT

(11) **EP 1 476 086 B1**
(45) Veröffentlichungstag und Bekanntmachung des Hinweises auf die Patenterteilung: **10.12.2008**
(21) Anmeldenummer: 03704497.1
(22) Anmeldetag: 30.01.2003
(51) Int. Cl.: A61B 17/16

(54) **CHIRURGISCHES GERÄT**
SURGICAL DEVICE
APPAREIL CHIRURGICAL

(30) Priorität: 19.02.2002 DE 10207355
(43) Veröffentlichungstag der Anmeldung: 17.11.2004
(73) Patentinhaber: Aesculap AG, 78532 Tuttlingen (DE)
(72) Erfinder: HÖGERLE, Roland, Alois, 78532 Tuttlingen (DE); MACHILL, Martin, 78604 Rietheim-Weilheim (DE); HÄUSLER, Rainer, 78532 Tuttlingen (DE)
(74) Vertreter: Hoeger, Stellrecht & Partner Patentanwälte
(86) Internationale Anmeldenummer: PCT/EP2003/000911
(87) Internationale Veröffentlichungsnummer: WO 2003/070113

(56) Entgegenhaltungen:
- EP-A- 0 261 260
- DE-A- 19 607 123
- DE-A- 19 953 772
- US-A- 5 571 633

## Beschreibung

Die Erfindung betrifft ein chirurgisches Gerät mit einem Gehäuse umfassend mindestens einen abgedichteten Hohlraum, mit einer in dem Gehäuse angeordneten Antriebseinheit.

Ein chirurgisches Gerät der eingangs beschriebenen Art ist beispielsweise aus der DE 196 07 123 A1 bekannt.

Bei der dort offenbarten Bohrmaschine ist vorgesehen, daß eine Batterie zusammen mit einer Steuerelektronik eines Elektromotors der Bohrmaschine in eine dafür vorgesehene Aufnahme im Handgriff des Geräts eingeführt und zum Sterilisieren des Geräts wieder entnommen werden kann. Eine Sterilisation der chirurgischen Geräte ist vor deren Einsatz in einem sterilen Operationsbereich notwendig. Allerdings kann für einzelne Bestandteile derartiger Geräte, insbesondere für elektrische Teile, eine üblicherweise durch eine Heißdampfbehandlung erfolgende Sterilisation bei erhöhter Temperatur schädlich sein. Bei der bekannten Bohrmaschine lassen sich so zwar Batterie und Steuerelektronik gegen schädliche Sterilisationseinflüsse schützen, jedoch besteht nach wie vor das Problem, daß beim Reinigen der Geräte in einem Waschprozeß mit Temperaturen des Reinigungsmittels von über 90°C abgedichtete Hohlräume nur idealerweise vollständig dicht sind und nur bedingt dauerhaft abgedichtet werden können.

Außerdem sind bei chirurgischen Geräten mit Antriebseinheiten stets Durchführungen, beispielsweise für eine Antriebswelle, erforderlich, deren dauerhafte und vollständige Abdichtung hohe Anforderungen an die Ausgestaltung und Ausführung erforderlicher Dichtverbindungen stellt. Vor allem beim Sterilisieren werden diese Dichtungen zusätzlich hohen mechanischen Belastungen ausgesetzt, da dabei große Druckdifferenzen zwischen dem Inneren des abgedichteten Hohlraumes und außerhalb desselben entstehen können. Als besonders nachteilig erweist sich dabei, daß stets geringe Mengen an Dampf oder Flüssigkeit in den abgedichteten Hohlraum unerwünschterweise eindringen können, aber nur schwer entfernbar sind. Damit besteht die Gefahr, daß auf dem umgekehrten Weg Keime aus dem Innern des abgedichteten Hohlraumes heraus in einen sterilen Operationsbereich gelangen können.

Dementsprechend ist es Aufgabe der vorliegenden Erfindung, ein chirurgisches Gerät der eingangs beschriebenen Art so zu verbessern, daß der Hohlraum auf einfache Weise abgedichtet und die Bildung von Keimen verringert bzw. diese auf einfache Weise aus dem Hohlraum entfernt werden können.

Diese Aufgabe wird bei einem chirurgischen Gerät der eingangs beschriebenen Art erfindungsgemäß dadurch gelöst, daß eine Fluidverbindung vorgesehen ist zum Ausbilden eines Strömungspfades in den Hohlraum hinein und aus diesem heraus und daß Dichtmittel vorgesehen sind zum Verschließen der Fluidverbindung für flüssige Fluide und zum Öffnen der Fluidverbindung für gasförmige Fluide.

Durch diese Ausgestaltung ist es auf einfache Weise möglich, den Hohlraum in seinem Inneren zu sterilisieren, denn durch die Fluidverbindung lassen sich gasförmige Fluide, beispielsweise Heißdampf ins Innere des Hohlraumes leiten und auf demselben Weg wieder daraus entfernen. Das Eindringen von Reinigungsflüssigkeit während eines Waschvorganges des Gerätes über die Fluidverbindung, z. B. in einer Spülmaschine, wird dagegen unterbunden. Da sich auf diese Weise das chirurgische Gerät gezielt von außen waschen und komplett, d. h. nicht nur außen, sondern auch innen sterilisieren läßt, ist eine Abdichtung des Hohlraumes nicht mehr mit dem bislang betriebenen Aufwand erforderlich. Somit ist es möglich, chirurgische Geräte, beispielsweise Bohrmaschinen oder Sägen, die jeweils eine Antriebseinheit aufweisen, vollständig keimfrei zu machen.

Günstig ist es, wenn die Dichtmittel thermisch aktivierbar sind zum Öffnen der Fluidverbindung für gasförmige Fluide für Temperaturen, die einer Aktivierungstemperatur entsprechen oder höhere Werte aufweisen. Damit wird ein Strömungspfad nur für Temperaturen ab der Aktivierungstemperatur geöffnet, beispielsweise auch automatisch. Unterhalb dieser Temperatur ist der Strömungspfad verschlossen, es können weder Flüssigkeiten noch Keime in das Innere des Gerätes eindringen oder aus diesem herausgelangen. Erst oberhalb der Aktivierungstemperatur, beispielsweise bei Beaufschlagung des Gerätes während des Sterilisiervorganges mit Heißdampf, der Temperaturen über 100° C aufweist, wird die Fluidverbindung für gasförmige Fluide geöffnet.

Vorzugsweise weist die Aktivierungstemperatur einen Wert von mindestens 120° C auf. Eine für einen Klinikbetrieb vorgeschriebene Sterilisation erfordert Temperaturen beim Sterilisiervorgang, die oberhalb von 120° C liegen. Werden diese Temperaturen nicht erreicht, ist die Fluidverbindung verschlossen.

Besonders vorteilhaft ist es, wenn die Dichtmittel druckabhängig aktivierbar sind zum Öffnen der Fluidverbindung für gasförmige Fluide bei Druckdifferenzen zwischen einem innerhalb des Hohlraumes herrschenden und einem außerhalb des Hohlraumes herrschenden Drucks, die einer Aktivierungsdifferenz entsprechen oder höhere Wert aufweisen. Damit wird verhindert, daß Fluide jedweder Art in das Innere des Hohlraumes eindringen können, falls die Aktivierungsdruckdifferenz unterhalb eines bestimmten Wertes liegt. Damit läßt sich die Fluidverbindung zum Sterilisieren des Geräts beispielsweise in Abhängigkeit der Prozeßbedingungen während des Sterilisiervorganges auch automatisch öffnen oder schließen. Es kann somit sichergestellt werden, daß nur unter bestimmten Druckverhältnissen gasförmige Fluide, beispielsweise Heißdampf in das Innere des Hohlraumes eindringen können.

Vorzugsweise beträgt die Aktivierungsdruckdifferenz mindestens 100 hPas. Dadurch wird verhindert, daß bei üblicherweise vorkommenden kleinen Druckschwankungen und damit verbundenen kleinen Druckdifferenzen zwischen dem Inneren und dem Äußeren des Hohlraumes, die Dichtmittel unbeabsichtigt geöffnet werden.

Ein besonders einfacher Aufbau ergibt sich, wenn die Dichtmittel eine Membran umfassen und wenn die Membran für gasförmige Fluide durchlässig und für flüssige Fluide undurchlässig ist. Auf diese Weise kann das Gerät in einer Reinigungsvorrichtung, beispielsweise einer Spülmaschine mit heißem oder kochendem Wasser beaufschlagt werden, ohne daß dasselbe oder etwaige darin enthaltene Reinigungs- und Desinfektionsmittel in das Innere des Hohlraumes gelangen und diesen verunreinigen können. Gleichzeitig ist es jedoch möglich, daß gasförmige Fluide, beispielsweise Heißdampf in das Innere des Hohlraumes zum Sterilisieren desselben gelangen können. Sich im Hohlraum z.B. durch Kondensation bildende Flüssigkeit kann durch Erwärmen des Gerätes und Verdampfen der Flüssigkeit wiederum durch die Membran hindurch aus dem Hohlraum entfernt werden. Somit läßt sich im Innenraum eine sterile und trockene Atmosphäre herstellen: Gleichzeitig ist über die als Membran ausgebildeten Dichtmittel ein Druckausgleich möglich, der Belastungen an Gehäuseteilen oder Dichtungen verringert oder ganz vermeidet.

Vorzugsweise sind die Dichtmittel aus einem Sinterwerkstoff hergestellt. Sinterwerkstoffe lassen sich auf einfache Weise herstellen und in ihrer Durchlässigkeit für bestimmte Moleküle gezielt einstellen. Beispielsweise kann auch eine Membran aus einem Sinterwerkstoff hergestellt sein.

Bei einer weiteren bevorzugten Ausführungsform der Erfindung kann vorgesehen sein, daß die Dichtmittel aus einem keramischen Werkstoff hergestellt sind. Auch Keramiken, die ebenfalls gesintert sein können, lassen sich in ihrer Durchlässigkeit für Fluide in gewünschter Weise herstellen.

Eine besonders kostengünstige und zuverlässige Ausgestaltung der Dichtmittel ergibt sich, wenn diese aus einem porösen oder geschäumten Kunststoffmaterial gebildet sind. So lassen sich beispielsweise atmungsaktive Membranen wie die unter den Markennamen Goretex^{®} und Sympatex^{®} vertriebenen Membranen als Dichtmittel einsetzen.

Vorteilhaft ist es, wenn die Dichtmittel eine mechanisch betätigbare Ventilanordnung mit mindestens einem Ventil umfassen. Dies eröffnet die Möglichkeit, die Fluidverbindung gezielt manuell zu öffnen oder zu schließen. Allerdings ist es auch denkbar, die mechanisch betätigbare Ventilanordnung aufgrund von bestimmten herrschenden Druckdifferenzen, oder in Abhängigkeit von herrschenden Temperaturen automatisch zu öffnen oder zu schließen.

Ein besonders einfacher Aufbau der Ventilanordnung ergibt sich, wenn das mindestens eine Ventil ein Membranventil ist.

Um den Sterilisiervorgang noch gezielter zu steuern, ist es günstig, wenn die Ventilanordnung ein Einströmventil und ein Ausströmventil umfaßt. Damit läßt sich die Beaufschlagung des Hohlraumes in seinem Innern mit Heißdampf bei beispielsweise anderen Temperaturen oder Druckdifferenzen durchführen als das Trocknen und/oder Entfeuchten des Hohlraumes.

Vorzugsweise weist das Einströmventil eine höhere Aktivierungsdruckdifferenz als das Ausströmventil auf. Auf diese Weise kann nur unter gezielten Bedingungen, beispielsweise beim Sterilisieren, ein Fluid in das Innere des Hohlraumes gelangen.

Der Aufbau der Ventilanordnung wird besonders einfach, wenn das Ventil ein Kugeldruckstück umfaßt.

Günstig ist es, wenn das mindestens eine Ventil von einem Bimetall betätigbar oder ein Bimetall ist. Damit läßt sich auf einfache Weise ein temperaturabhängig betätigbares Ventil bilden, das beispielsweise beim Erreichen und Überschreiten einer Aktivierungstemperatur die Fluidverbindung öffnet.

Besonders einfach läßt sich eine Fluidverbindung herstellen, wenn sie eine Bohrung umfaßt.

Um ein unerwünschten Eindringen von Fluiden in das Innere des Hohlraumes zusätzlich zu erschweren, ist vorgesehen, daß die Fluidverbindung einen labyrinthartigen Kanal umfaßt.

Grundsätzlich kann jedes chirurgische Gerät, welches eine Antriebseinheit umfaßt, mit einer erfindungsgemäß ausgestalteten Fluidverbindung versehen sein. Vorzugsweise ist das chirurgische Gerät jedoch eine chirurgische Bohrmaschine oder eine chirurgische Säge.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht einer chirurgischen Handbohrmaschine und
- Figur 2:: eine vergrößerte Ansicht des Bereichs A in Figur 1.

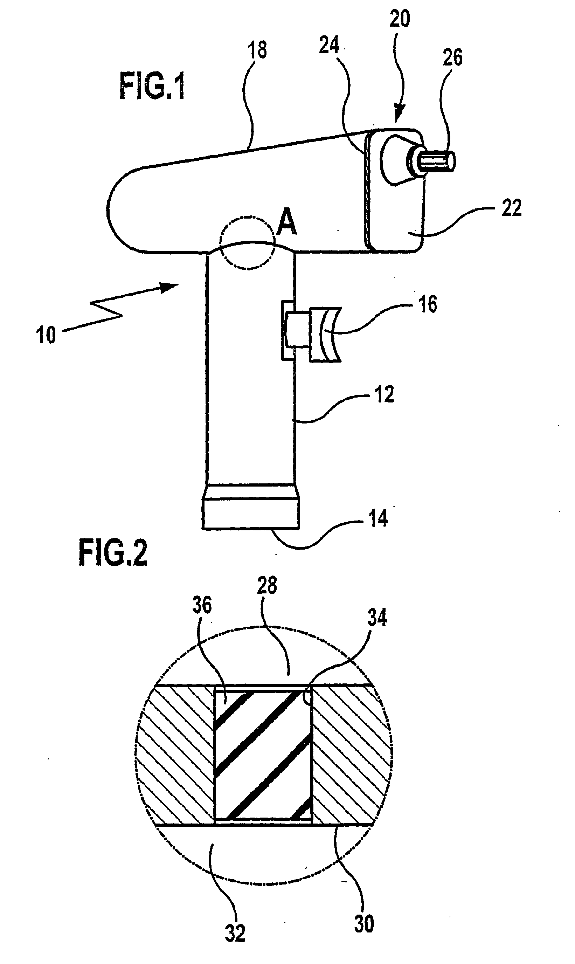

In Figur 1 ist eine insgesamt mit dem Bezugszeichen 10 versehene chirurgische Handbohrmaschine dargestellt, die von einer Batterie oder einem Akkumulator betrieben wird, welche in eine Batterieaufnahme 32 eines mit einem Deckel 14 verschließbaren hohlen Handgriffs 12 der Handbohrmaschine 10 eingesetzt werden können. Zum Betreiben der Handbohrmaschine 10 ist ein quer zu einer Längsrichtung des Handgriffs 12 bewegbarer Schaltknopf 16 angeordnet, der mit der Batterie und einer mit dieser verbundenen Steuerelektronik in Eingriff bringbar ist.

Quer zum Handgriff 12 ist ein langgestrecktes Motorgehäuse 18 angeordnet, dessen eine Stirnseite 20 mit einem Gehäusedeckel 22 verschlossen ist, wobei zwischen dem Gehäusedeckel 22 und dem Motorgehäuse 18 eine Dichtung 24 eingesetzt ist. Durch den Gehäusedeckel 22 ist eine von einem in dem Motorgehäuse 18 angeordneten, nicht dargestellten Motor angetriebene Welle 26 herausgeführt, die mit Bohrwerkzeugen verbunden werden kann.

Das Motorgehäuse 18 umschließt einen insgesamt vollständig abgedichteten Hohlraum 28. Eine Trennwand 30, die den Hohlraum 28 von der Batterieaufnahme 32 trennt, wird von einer Bohrung 34 durchsetzt. Diese wiederum ist mit einer Membran 36 verschlossen, die für gasförmige Fluide durchlässig ist, jedoch die Bohrung 34 für flüssige Fluide vollständig verschließt. Die Membran 36 kann aus einer Keramik, einem porösen oder geschäumten Kunststoffmaterial oder aus einem Sinterwerkstoff, beispielsweise aus einem Metall, hergestellt sein. Alternativ wäre es auch denkbar, in die Bohrung 34 ein mechanisch arbeitendes Ventil, beispielsweise ein Kugeldruckstück oder ein Bimetall einzusetzen. Auch ein "Membranventil, das die Bohrung 34 verschließt, wäre denkbar. Statt der Bohrung 34 könnte alternativ ein labyrinthartiger Kanal vorgesehen sein, der mit den im Zusammenhang mit der Bohrung 34 beschriebenen und in diese eingesetzten Dichtelemente verschlossen werden kann.

## Patentansprüche

1. Chirurgisches Gerät mit einem Gehäuse umfassend mindestens einen abgedichteten Hohlraum, mit einer in dem Gehäuse angeordneten Antriebseinheit, **dadurch gekennzeichnet, daß** eine Fluidverbindung (34) vorgesehen ist zum Ausbilden eines Strömungspfades in den Hohlraum (28) hinein und aus diesem heraus und daß Dichtmittel (36) vorgesehen sind zum Verschließen der Fluidverbindung (34) für flüssige Fluide und zum Öffnen der Fluidverbindung (34) für gasförmige Fluide.

2. Gerät nach Anspruch 1, **dadurch gekennzeichnet, daß** die Dichtmittel (36) thermisch aktivierbar sind zum Öffnen der Fluidverbindung (34) für gasförmige Fluide für Temperaturen, die einer Aktivierungstemperatur entsprechen oder höhere Werte aufweisen.

3. Gerät nach Anspruch 2, **dadurch gekennzeichnet, daß** die Aktivierungstemperatur einen Wert von mindestens 120° Celsius aufweist.

4. Gerät nach einem der voranstehenden Ansprüche, **dadurch gekennzeichnet, daß** die Dichtmittel (36) druckabhängig aktivierbar sind zum Öffnen der Fluidverbindung (34) für gasförmige Fluide bei Druckdifferenzen zwischen einem innerhalb des Hohlraums (28) herrschenden und einem außerhalb des Hohlraums (28) herrschenden Drucks, die einer Aktivierungsdruckdifferenz entsprechen oder höhere Werte aufweisen.

5. Gerät nach Anspruch 4, **dadurch gekennzeichnet, daß** die Aktivierungsdruckdifferenz mindestens 100 hPas beträgt.

6. Gerät nach einem der voranstehenden Ansprüche, **dadurch gekennzeichnet, daß** die Dichtmittel eine Membran (36) umfassend und daß die Membran (36) für gasförmige Fluide durchlässig und für flüssige Fluide undurchlässig ist.

7. Gerät nach einem der voranstehenden Ansprüche, **dadurch gekennzeichnet, daß** die Dichtmittel (36) aus einem Sinterwerkstoff hergestellt sind.

8. Gerät nach einem der voranstehenden Ansprüche, **dadurch gekennzeichnet, daß** die Dichtmittel (36) aus einem keramischen Werkstoff hergestellt sind.

9. Gerät nach einem der voranstehenden Ansprüche, **dadurch gekennzeichnet, daß** die Dichtmittel (36) aus einem porösen oder geschäumten Kunststoffmaterial gebildet sind.

10. Gerät nach einem der voranstehenden Ansprüche, **dadurch gekennzeichnet, daß** die Dichtmittel (36) eine mechanisch betätigbare Ventilanordnung mit mindestens einem Ventil umfassen.

11. Gerät nach Anspruch 10, **dadurch gekennzeichnet, daß** das mindestens eine Ventil ein Membranventil ist.

12. Gerät nach einem der Ansprüche 10 oder 11, **dadurch gekennzeichnet, daß** die Ventilanordnung ein Einströmventil und ein Ausströmventil umfaßt.

13. Gerät nach Anspruch 12, **dadurch gekennzeichnet, daß** das Einströmventil eine höhere Aktivierungsdruckdifferenz als das Ausströmventil aufweist.

14. Gerät nach einem der Ansprüche 10 bis 13, **dadurch gekennzeichnet, daß** das mindestens eine Ventil ein Kugeldruckstück umfaßt.

15. Gerät nach einem der Ansprüche 10 bis 14, **dadurch gekennzeichnet, daß** das mindestens eine Ventil von einem Bimetall betätigbar oder ein Bimetall ist.

16. Gerät nach einem der voranstehenden Ansprüche, **dadurch gekennzeichnet, daß** die Fluidverbindung eine Bohrung (34) umfaßt.

17. Gerät nach Anspruch 5, **dadurch gekennzeichnet, daß** die Fluidverbindung (34) einen labyrinthartigen Kanal umfaßt.

18. Gerät nach einem der voranstehenden Ansprüche, **dadurch gekennzeichnet, daß** das chirurgische Gerät eine chirurgische Bohrmaschine (10) oder eine chirurgische Säge ist.

## Claims

1. Surgical appliance with a housing comprising at least one sealed cavity, with a drive unit arranged in the housing, **characterized in that** a fluid connection (34) is provided for formation of a flow path into and out of the cavity (28), and **in that** sealing means (36) are provided for closing the fluid connection (34) to liquid fluids and opening the fluid connection (34) to gaseous fluids.

2. Appliance in accordance with claim 1, **characterized in that** the sealing means (36) are thermally activatable for opening the fluid connection (34) to gaseous fluids at temperatures which correspond to an activation temperature or have higher values.

3. Appliance in accordance with claim 2, **characterized in that** the activation temperature has a value of at least 120° Celsius.

4. Appliance in accordance with any one of the preceding claims, **characterized in that** the sealing means (36) are activatable in a pressure-dependent manner for opening the fluid connection (34) to gaseous fluids at pressure differences between a pressure prevailing inside the cavity (28) and a pressure prevailing outside the cavity (28), which correspond to an activation pressure difference or have higher values.

5. Appliance in accordance with claim 4, **characterized in that** the activation pressure difference is at least 100 hPas.

6. Appliance in accordance with any one of the preceding claims, **characterized in that** the sealing means comprise a membrane (36), and **in that** the membrane (36) is permeable to gaseous fluids and impermeable to liquid fluids.

7. Appliance in accordance with any one of the preceding claims, **characterized in that** the sealing means (36) are made of a sintered material.

8. Appliance in accordance with any one of the preceding claims, **characterized in that** the sealing means (36) are made of a ceramic material.

9. Appliance in accordance with any one of the preceding claims, **characterized in that** the sealing means (36) are made of a porous or foamed plastic material.

10. Appliance in accordance with any one of the preceding claims, **characterized in that** the sealing means (36) comprise a mechanically actuatable valve assembly with at least one valve.

11. Appliance in accordance with claim 10, **characterized in that** the at least one valve is a diaphragm valve.

12. Appliance in accordance with claim 10 or 11, **characterized in that** the valve assembly comprises an intake valve and a discharge valve.

13. Appliance in accordance with claim 12, **characterized in that** the intake valve has a higher activation pressure difference than the discharge valve.

14. Appliance in accordance with any one of claims 10 to 13, **characterized in that** the at least one valve comprises a ball thrust member.

15. Appliance in accordance with any one of claims 10 to 14, **characterized in that** the at least one valve is actuatable by a bimetal or is a bimetal.

16. Appliance in accordance with any one of the preceding claims, **characterized in that** the fluid connection comprises a bore (34).

17. Appliance in accordance with claim 5, **characterized in that** the fluid connection (34) comprises a labyrinth-type channel.

18. Appliance in accordance with any one of the preceding claims, **characterized in that** the surgical appliance is a surgical drill (10) or a surgical saw.

## Revendications

1. Appareil chirurgical comportant un boîtier comprenant au moins une cavité étanchée, comportant une unité d'entraînement agencée dans le boîtier, **caractérisé en ce qu'**il est prévu une liaison fluidique (34) pour réaliser un chemin d'écoulement jusqu'à l'intérieur de la cavité (28) et hors de celle-ci, et **en ce qu'**il est prévu des moyens d'étanchéité (36) pour fermer la liaison fluidique (34) pour des fluides liquides et pour ouvrir la liaison fluidique (34) pour des fluides gazeux.

2. Appareil selon la revendication 1, **caractérisé en ce que** les moyens d'étanchéité (36) peuvent être activés thermiquement pour ouvrir la liaison fluidique (34) pour des fluides gazeux pour des températures qui correspondent à une température d'activation ou qui présentent des valeurs plus élevées.

3. Appareil selon la revendication 2, **caractérisé en ce que** la température d'activation présente une valeur d'au moins 120°C.

4. Appareil selon l'une quelconque des revendications précédentes, **caractérisé en ce que** les moyens d'étanchéité (36) peuvent être activés en fonction de la pression pour ouvrir la liaison fluidique (34) pour des fluides gazeux en cas de différences de pression entre une pression régnant à l'intérieur de la cavité (28) et une pression régnant à l'extérieur de la cavité (28), qui correspondent à une différence de pression d'activation ou qui présentent des valeurs plus élevées.

5. Appareil selon la revendication 4, **caractérisé en ce que** la différence de pression d'activation est d'au moins 100 hPa.

6. Appareil selon l'une quelconque des revendications précédentes, **caractérisé en ce que** les moyens d'étanchéité comprennent une membrane (36) et **en ce que** la membrane (36) est perméable aux fluides gazeux et imperméable aux fluides liquides.

7. Appareil selon l'une quelconque des revendications précédentes, **caractérisé en ce que** les moyens d'étanchéité (36) sont réalisés en un matériau fritté.

8. Appareil selon l'une quelconque des revendications précédentes, **caractérisé en ce que** les moyens d'étanchéité (36) sont réalisés en un matériau céramique.

9. Appareil selon l'une quelconque des revendications précédentes, **caractérisé en ce que** les moyens d'étanchéité (36) sont formés en une matière plastique poreuse ou moussée.

10. Appareil selon l'une quelconque des revendications précédentes, **caractérisé en ce que** les moyens d'étanchéité (36) comprennent un agencement de soupape à actionnement mécanique avec au moins une soupape.

11. Appareil selon la revendication 10, **caractérisé en ce que** ladite au moins une soupape est une soupape à membrane.

12. Appareil selon l'une quelconque des revendications 10 ou 11, **caractérisé en ce que** l'agencement de soupapes présente une soupape d'admission et une soupape d'échappement.

13. Appareil selon la revendication 12, **caractérisé en ce que** la soupape d'admission présente une différence de pression d'activation supérieure à la soupape d'échappement.

14. Appareil selon l'une des revendications 10 à 13, **caractérisé en ce que** ladite au moins une soupape comprend une pièce à bille recevant la pression.

15. Appareil selon l'une des revendications 10 à 14, **caractérisé en ce que** ladite au moins une soupape peut être actionnée par un bilame ou est un bilame.

16. Appareil selon l'une quelconque des revendications précédentes, **caractérisé en ce que** la liaison fluidique comprend un perçage (34).

17. Appareil selon la revendication 5, **caractérisé en ce que** la liaison fluidique (34) comprend un canal en forme de labyrinthe.

18. Appareil selon l'une quelconque des revendications précédentes, **caractérisé en ce que** l'appareil chirurgical est une perceuse (10) chirurgicale ou une scie chirurgicale.
